# EUROPEAN PATENT APPLICATION

(11) **EP 2 298 882 A1**
(43) Date of publication of application: **23.03.2011**
(21) Application number: 09754466.2
(22) Date of filing: 29.05.2009
(51) Int. Cl.: C12N 15/09, C12N 1/21, C12N 7/02

(54) **RECOMBINANT VIRUS, ESCHERICHIA COLI RETAINING THE SAME AND A PROCESS FOR PRODUCTION THEREOF**

(30) Priority: 30.05.2008 JP 2008142662
(71) Applicant: The University of Tokyo, Bunkyo-Ku Tokyo 113-8654 (JP)
(72) Inventor: KAWAGUCHI, Yasushi, Tokyo 113-8654 (JP); MORIMOTO, Tomomi, Tokyo 113-8654 (JP)
(74) Representative: Blodig, Wolfgang
(86) International application number: PCT/JP2009/002394
(87) International publication number: WO 2009/144958

(57) **Abstract**

To provide a method for producing a recombinant virus retaining the characteristics of the genome of a target virus.

The method for producing a recombinant virus including inserting a foreign gene into a region between polyA signals of two genes encoded in directions opposing one another in the genome of a target virus.

## Description

### Technical Field

The present invention relates to a method for producing a recombinant virus useful for, for example, development of vaccines or gene therapy vectors; to a recombinant virus retaining the full-length genome of herpes simplex virus type 2; and to *Escherichia coli* including the recombinant virus.

### Background Art

For production of a recombinant virus such as a gene therapy vector or a multivalent vaccine, a foreign gene must be inserted into any region of the genome of a virus of interest. The thus-produced recombinant virus poses problems, when gene deletion occurs in the genome of the original virus, or when a change occurs in a property of the virus for a gene therapy vector or a vaccine.

Conventionally known techniques for producing recombinant viruses include homologous recombination in cultured cells by use of a marker (TK or LacZ) gene (Non-Patent Document 1), and homologous recombination in cultured cells by use of a cosmid (Non-Patent Document 2). Each of these conventional techniques is focused on a foreign gene which is to be inserted into the genome of a virus of interest, but is not focused on the genome of the virus into which the foreign gene is to be inserted.

Herpes simplex virus (HSV) infection is caused by HSV-1 or HSV-2. HSV-1 generally infects the upper part of a body, in particular, the oral cavity or upper respiratory tract mucosa, to thereby develop stomatitis or herpes labialis. Meanwhile, HSV-2 generally infects the lower part of a body, in particular, genitalia, to thereby develop genital herpes. No HSV vaccine has yet been developed. HSV is useful as a gene therapy vector, and some HSV vectors are under clinical trial.

Development of virus vaccines or gene therapy vectors requires a technique for producing a recombinant virus. Development of a vaccine requires inactivation of a pathogenic factor, and development of a gene therapy vector requires incorporation of a foreign gene in addition to inactivation of a pathogenic factor.

In recent years, there has been reported a technique for homologous recombination in *Escherichia coli* making use of the bac system (bacterial artificial chromosome system), which is a large DNA cloning system. Thus, production of recombinant viruses has been more rapid and convenient than ever before.

The key material in the bac system is *Escherichia coli* including the genome of a virus of interest. When such *Escherichia coli* can be produced, various modifications can be performed in the virus genome by utilizing the genetics of the *Escherichia coli.* In the bac system, insertion of bacmid into the genome of a is required for *Escherichia coli* to include the genome of the virus, but problems arise when, through bacmid insertion, gene deletion occurs in the virus genome, or a property of the wild type of the virus changes. However, as has been conventionally reported, in many cases of production of *Escherichia coli* including the genome of HSV by means of the bac system, deletion of a packaging signal occurs through insertion of bacmid (Non-Patent Documents 3 to 5), or bacmid is inserted into TK (non-essential HSV gene) (Non-Patent Document 6). The presence of such deletion or insertion may be inappropriate for production of a multipurpose vector, and also may be impeditive for use thereof in fundamental studies of the virus itself. Since bacmid has a relatively large size (i.e., 7 kbp), a limitation is imposed on the pathogenicity of a recombinant virus, or on the length of a foreign gene which can be retained by the recombinant virus. Therefore, a system for removing bacmid is required.

It was previously reported that there can be successfully produced *Escherichia coli* including the full-length genome of infectious HSV-1 with its wild-type property being retained, by inserting bacmid into a region between the UL3 and UL4 genes of HSV-1 (Patent Document 1)

However, there has not yet been produced *Escherichia coli* which can include a recombinant virus in which the full-length genome of HSV-2 is substantially retained.

### Prior Art Document

### Patent Document

Patent Document 1: JP-A-2003-189882

### Non-Patent Document

Non-Patent Document 1: Post L. E. et al., Cell 24: 555-565, 1981
Non-Patent Document 2: Cunningham C. et al., Virol. 197: 116-124, 1993
Non-Patent Document 3: Saeki Y. et al., Hum. Gen. Ther. 9: 2787-2794, 1998
Non-Patent Document 4: Tom A. S. et al., J. Virol. 72: 7137-7143, 1998
Non-Patent Document 5: Suter M. et al., Proc. Natl. Acad. Sci. USA 96: 12697-12702, 1999
Non-Patent Document 6: Horsburgh B. C. et al., Gene Ther. 6: 922-930, 1999

### Summary of the Invention

### Problems to be Solved by the Invention

An object of the present invention is to provide a method for inserting a foreign gene into the genome of a virus of interest without causing gene deletion in the genome of the virus or a change in property of the virus.
Another object of the present invention is to provide an HSV-2 recombinant retaining the full-length genome of HSV-2. Yet another object of the present invention is to provide *Escherichia coli* including the genome of the recombinant virus.

### Means for Solving the Problems

The present inventors have conducted extensive studies for producing a recombinant virus by inserting a foreign gene into a target virus, without causing gene deletion in the genome of the target virus and without affecting a property of the target virus. As a result, the present inventors have found that when a foreign gene is inserted into a region between polyA signals of genes encoded in directions opposing one another in the genome of a target virus, a recombinant virus of interest can be readily produced. The present inventors have also found that when bacmid is inserted into a region between polyA signals of the UL50 and UL51 genes of HSV-2, an HSV-2 recombinant which substantially retains the full-length genome of HSV-2 can be produced. The present invention has been accomplished by attaining *Escherichia coli* including the genome of the recombinant virus.

Accordingly, the present invention provides a method for producing a recombinant virus, including inserting a foreign gene into a region between polyA signals of two genes encoded from opposite directions in the genome of a target virus, excepting a region between the UL3 and UL4 genes of HSV-1.
The present invention also provides a method for producing *Escherichia coli* including the genome of a recombinant virus which substantially retains the full-length genome of a wild-type virus, the method includes inserting bacmid into a region between polyA signals of two genes encoded from directions opposing one another in the genome of a target virus, excepting a region between the UL3 and UL4 genes of HSV-1, and incorporating the genome of the resultant recombinant virus into *Escherichia coli.*
The present invention also provides an HSV-2 recombinant which substantially retains the full-length genome of wild-type HSV-2, and in which is inserted into a region between the UL50 and UL51 genes.
The present invention also provides *Escherichia coli* including the genome of an HSV-2 recombinant which substantially retains the full-length genome of wild-type HSV-2, and in which bacmid is inserted into a region between the UL50 and UL51 genes.

### Effects of the Invention

According to the method of the present invention, there can be readily produced a recombinant virus which is useful as a tool for developing vaccines or gene therapy vectors. When bacmid is employed as a foreign gene, *Escherichia coli* including the genome of the recombinant virus is produced. Since the thus-produced *Escherichia coli* allows remarkably easy modification of a virus, employment of the *Escherichia coli* realizes development of vaccines or gene therapy vectors.
The HSV-2 recombinant of the present invention substantially retains the full-length genome of wild-type HSV-2, and thus it is useful for vectors for gene expression or development of vaccines and fundamental studies thereon.

### Brief Description of the Drawings

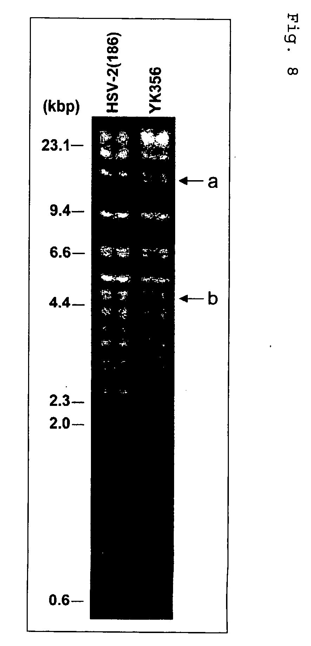

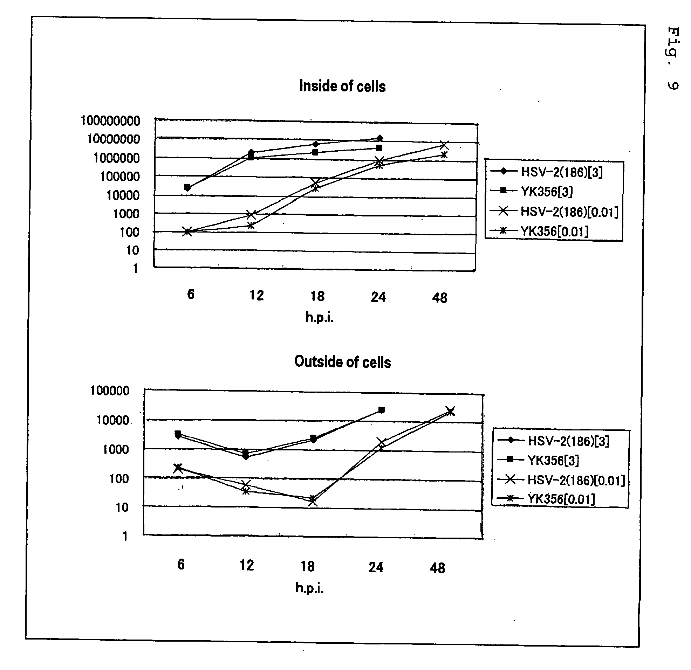

[Fig. 1]
   A region between polyA signals, which is a gene insertion site.
[Fig. 2]
   Processes for constructing YK336, YK338, and YK339.
[Fig. 3]
   Processes for constructing YK381 and YK382.
[Fig. 4]
   Graphs showing the growth potentials of HSV-1, YK336, YK338, and YK339.
[Fig. 5]
   Graphs showing the growth potentials of HSV-2, YK381, and YK382.
[Fig. 6]
   Process for constructing pBS-2NC-GFP/BAC.
[Fig. 7]
   Processes for constructing YK351 and YEbac356.
[Fig. 8]
   Restriction enzyme cleavage pattern of YK356 (represented as BAC in the figure). In Fig. 8, HSV-2(186) corresponds to a wild-type strain.
[Fig. 9]
   Graphs showing the growth potentials of YK356 (represented as BAC in the figure) and wild-type HSV-2 186 (represented as 186 in the figure). In Fig. 9, the values in [ ] correspond to MOI (multiplicity of infection). The upper graph shows the amount of a virus present inside of cells infected with the virus, and the lower graph shows the amount of a virus present outside of cells infected with the virus.
[Fig. 10]
   A graph showing the results of mouse infection experiments (survival curves). BAC corresponds to YK356, and 186 corresponds to a wild-type strain.
[Fig. 11]
   Process for constructing YK361.
[Fig. 12]
   Results of Southern blotting of YK801 and YK356.
[Fig. 13]
   Results of point mutation of a wild-type strain (wt) and Us3 mutation (US3-KM).

### Best Modes for Carrying Out the Invention

Now will be described a method for inserting a foreign gene into a specific site of the genome of a target virus.
In the method of the present invention, a foreign gene is inserted into a region between polyA signals of two genes encoded in directions opposing one another in the genome of a target virus. No particular limitation is imposed on the target virus, so long as it is in the form of DNA. Examples of the target virus include herpesvirus, adenovirus, poxvirus, and papovavirus.

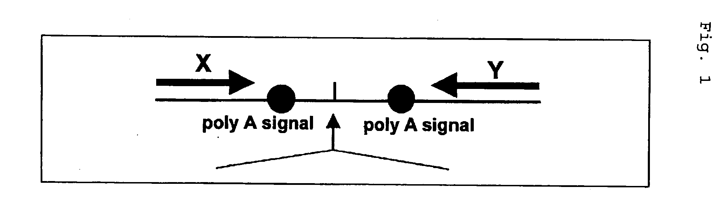

As used herein, "region between polyA signals of two genes encoded in directions opposing one another in the genome of a target virus" refers to a region between polyA signals present in a region of two genes which are adjacent and encoded in directions opposing one another in the genome (see Fig. 1). Such a region can be selected by reference to the database of the genome of a target virus. For example, in the case of HSV-2, such a region corresponds to the following 13 regions: the UL3-UL4 region, the UL7-UL8 region, the UL10-UL11 region, the UL15-UL18 region, the UL21-UL22 region, the UL26-UL27 region, the UL35-UL36 region, the UL40-UL41 region, the UL45-UL46 region, the UL50-UL51 region, the UL55-UL56 region, the Us1-Us2 region, and the Us9-Us10 region.
When a foreign gene is inserted into a region between two genes encoded in the same direction in the genome of a target virus, a promoter may be disrupted. However, when a foreign gene is inserted into a region between polyA signals as in the case of the method of the present invention, no gene deletion occurs in the genome of a target virus, and no change occurs in a property of the virus.

Examples of the foreign gene employed include bacmid, a reporter gene, a gene encoding an immunogenic protein, and a gene used for gene therapy. When the genome of a recombinant virus is intended to be included in *Escherichia coli,* bacmid is preferably employed. For example, there may be employed a vector including bacmid which is sandwiched between two loxP sites, or a vector including bacmid and a detectable reporter gene which are sandwiched between two loxP sites. Examples of the detectable reporter gene employed include GFP (green fluorescent protein), CAT (chloramphenicol acetyltransferase), DsRed, GUS (β-glucuronidase), lacZ, Kaede, and luciferase. Of these, GFP is particularly preferred.

Insertion of a foreign gene into a region between polyA signals may be carried out through customary cloning means employing a restriction enzyme site. For example, a fragment including the region between polyA signals, which is an insertion site of interest in the genome of a target virus, is cloned into a plasmid. A foreign gene (and optionally a reporter gene) sandwiched between two loxP sites is inserted into the region between the two polyA signals in the resultant plasmid, to thereby produce a plasmid including the foreign gene inserted into the region between the polyA signals.

Subsequently, the DNA of the target virus and the foreign-gene-inserted plasmid are transfected into cells which can be infected with the target virus, followed by homologous recombination, to thereby produce a recombinant virus of interest. The recombinant virus of interest can be selected by, for example, selecting, from the thus-infected cells, cells in which the reporter gene is expressed.

When the thus-produced recombinant virus includes bacmid, a bacterium including the recombinant virus can be produced by incorporating the genome of the recombinant virus into *Escherichia coli* or a similar bacterium.

Specifically, virus-infectable cells such as Vero cells or rabbit skin cells are infected with the resultant recombinant virus; circular virus DNA is collected from the thus-infected cells; and the circular virus DNA is incorporated into *Escherichia coli.*

Next will be described a method for producing the HSV-2 recombinant of the present invention. The HSV-2 recombinant of the present invention is produced by using bacmid as a foreign gene and the UL50-UL51 region as a region between polyA signals. An attempt was made to insert bacmid into the UL3-UL4 region of HSV-2, but this attempt resulted in failure. Therefore, the UL50-UL51 region is preferably employed for insertion of bacmid into HSV-2. Firstly, a plasmid including a fragment having the UL50-UL51 region is constructed. Bacmid is inserted into the UL50-UL51 region of the plasmid. Specifically, bacmid sandwiched between two loxP sequences, or bacmid and a reporter gene (e.g., GFP) sandwiched between two loxP sequences are inserted into the UL50-UL51 region of the plasmid by use of a plasmid including bacmid sandwiched between two loxP sequences, or a plasmid including bacmid and the reporter gene sandwiched between two loxP sequences.
Subsequently, the resultant recombinant plasmid and the DNA of wild-type HSV-2 are transfected into cells which can be infected with HSV-2, followed by homologous recombination, to thereby produce an HSV-2 recombinant which substantially retains the full-length genome of wild-type HSV-2 and in which bacmid is inserted into the UL50-UL51 intergenic region. As used herein, "substantially" refers to the case where the HSV-2 recombinant retains the full-length genome of wild-type HSV-2 without causing any damage to the function of the virus.

When the genome of the thus-produced HSV-2 recombinant is incorporated into *Escherichia coli, Escherichia coli* including the genome of the HSV-2 recombinant is obtained.

Since the thus-produced HSV-2 recombinant includes loxP sites, bacmid or both bacmid and the reporter gene can be removed by use of Cre recombinase.

The thus-produced HSV-2 recombinant, or an HSV-2 recombinant reconstructed from the genome of the HSV-2 recombinant included in *Escherichia coli* exhibits growth potential and pathogenicity comparable to those of wild-type HSV-2, and can be employed as a platform of a virus vector or a vaccine.

### Examples

The present invention will next be described in detail by way of examples, which should not be construed as limiting the invention thereto.

In the below-described Examples, gene cloning and analysis thereof were carried out according to Maniates, et al., Molecular Cloning-A Laboratory Manual (Cold Spring Harbor Laboratory) (1982). Experiments were carried out according to a standard laboratory procedure as described in detail in Ausubel F. *et al*., Current Protocols in Molecular Biology (1987).

### Example 1

### (Method for producing recombinant virus including fluorescent protein cassette inserted into intergenic region)

### (1) Construction of transfer plasmid

For production of p26.5-Venus, a UL26.5 promoter region of HSV-1(F) (nt 51388 to nt 51673), Venus fluorescent protein gene, and a 270 bp fragment including bidirectional polyA signals of UL21 and UL22 of HSV-1 were cloned into pBluescript II KS+ (Stratagene). An SacI-KpnI fragment of p26.5-Venus was cloned into a BamHI site of pRB442 (J. Virol. 65: 938-944, 1991), to thereby produce a transfer plasmid p26.5-Venus in UL3-4. A BglII-EcoRI fragment of HSV-1(F) including UL50 and UL51 (nt 106750 to nt 110095) was cloned into pBluescript II KS+, and a PacI site was incorporated into a region between polyA signals of the UL50 and UL51 of the resultant plasmid, to thereby produce a plasmid pUL50-51pac. An SacI-KpnI fragment of p26.5-Venus was cloned into the PacI site of the pUL50-51pac, to thereby produce a transfer plasmid p26.5-Venus in UL50-51. An EcoRI-BamHI fragment of HSV-1(F) including Us1 and Us2 (nt 131535 to nt 136289) was cloned into pBluescript II KS+, and a PacI site was incorporated into a region between polyA signals of the Us1 and Us2 of the resultant plasmid, to thereby produce a plasmid pUs1-2pac. An SacI-KpnI fragment of p26.5-Venus was cloned into the PacI site of the pUs1-2pac, to thereby produce a transfer plasmid p26.5-Venus in Us1-2.
A BamHI fragment of the genome of HSV-2 186 virus including the UL3 and UL4 genes (nt 6772 to nt 14950) was cloned into pBluescript II KS+, and a PacI site was incorporated into a region between polyA signals of the UL3 and UL4 of the resultant plasmid, to thereby produce a plasmid p2UL3-4pac. An SacI-KpnI fragment of p26.5-Venus was cloned into the PacI site of the p2UL3-4pac, to thereby produce a transfer plasmid p26.5-Venus in 2UL3-4. An NotI-ClaI fragment of the genomic DNA of HSV-2 186 strain including the UL50 and UL51 genes (n.n. 106473 to n.n. 110443) was cloned into an NotI-ClaI site of pBluescript II KS+ (Stratagene), to thereby produce pBS-2NC. An SacI-KpnI fragment of p26.5-Venus was cloned into an NdeI site in a region between polyA signals of the UL50 and UL51 genes of pBS-2NC, to thereby produce a transfer plasmid p26.5-Venus in 2UL50-51.

### (2) Production of recombinant virus

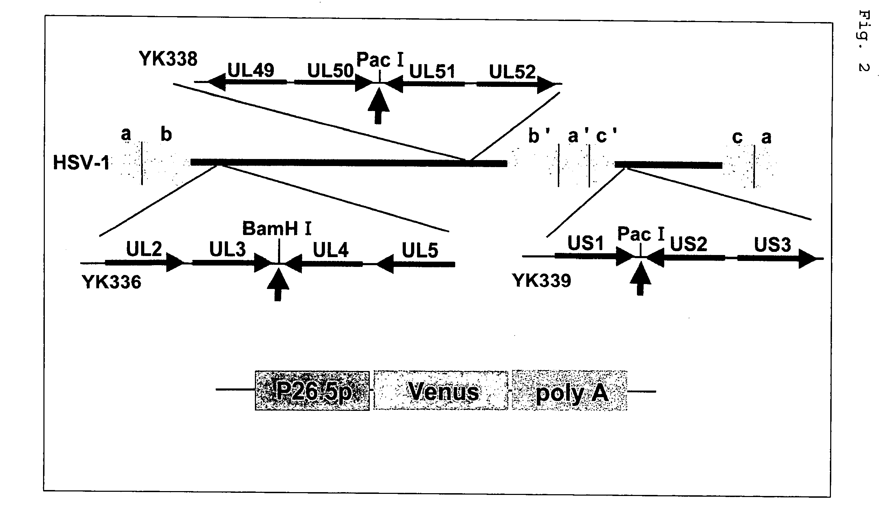

The virus DNA of HSV-1(F) strain and p26.5-Venus in UL3-4, p26.5-Venus in UL50-51, or p26.5-Venus in Us1-2 were transfected into rabbit skin cells (RSC), followed by homologous recombination, to thereby produce a recombinant virus including a Venus expression cassette inserted into an intergenic region. The thus-produced recombinant virus YK336 (insertion into UL3-UL4 intergenic region), YK338 (insertion into UL50-UL51 intergenic region), or YK339 (insertion into Us1-Us2 intergenic region) (Fig. 2) was selected by collecting a fluorescent plaque under a fluorescence microscope. Plaque purification was carried out under a fluorescence microscope until 100% of the plaque fluoresced. YK336, YK338, or YK339 was confirmed to be a recombinant virus of interest through Southern blotting.

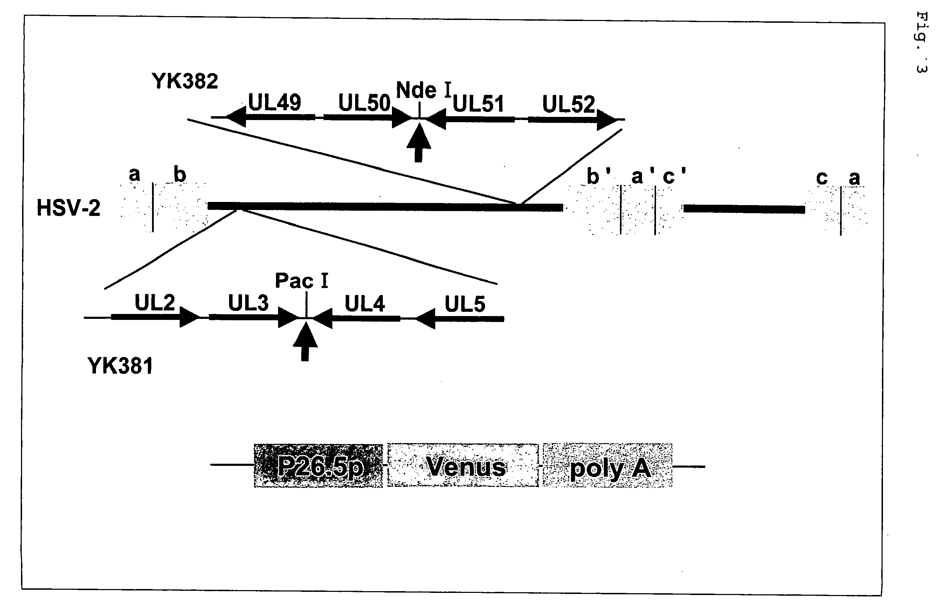

The virus DNA of HSV-2 186 strain and p26.5-Venus in 2UL3-4 or p26.5-Venus in 2UL50-51 were transfected into rabbit skin cells (RSC), followed by homologous recombination, to thereby produce a recombinant virus including a Venus expression cassette inserted into an intergenic region. The thus-produced recombinant virus YK381 (insertion into UL3-UL4 intergenic region) or YK382 (insertion into UL50-UL51 intergenic region) (Fig. 3) was selected by collecting a fluorescent plaque under a fluorescence microscope. Plaque purification was carried out under a fluorescence microscope until 100% of the plaque fluoresced. YK381 or YK382 was confirmed to be a recombinant virus of interest through Southern blotting.

### (3) Analysis of property of recombinant virus

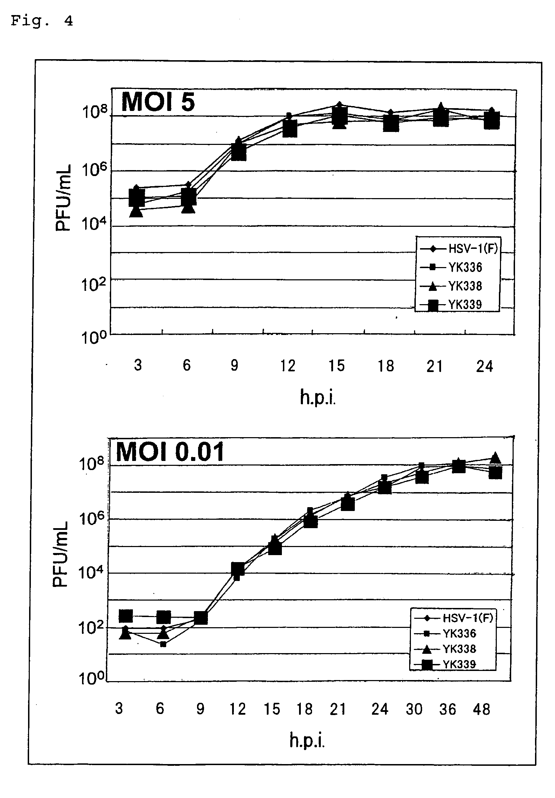

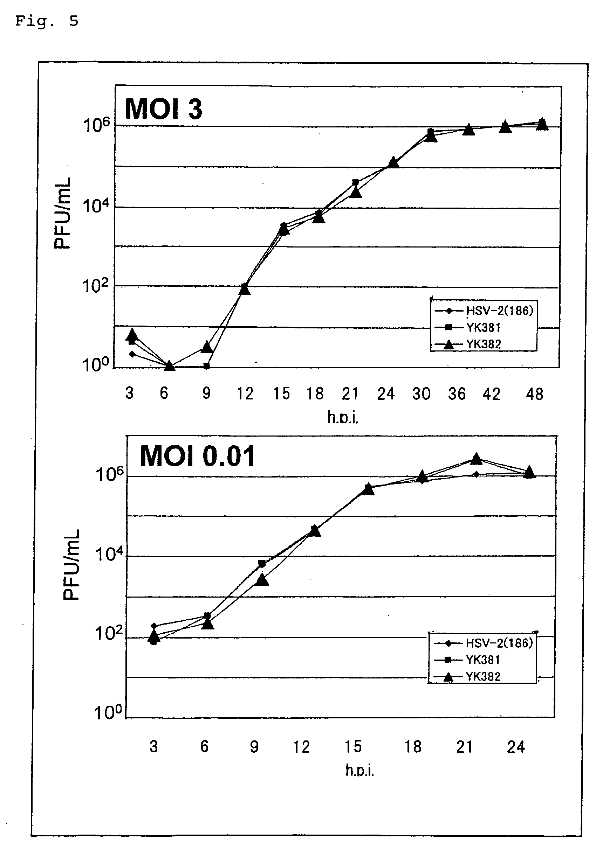

Vero cells were infected with YK336, YK338, YK339, or wild-type HSV-1(F) at MOI 0.01 or MOI 5, and the amount of the virus present inside and outside of the virus-infected cells was determined for comparison of growth potential (Fig. 4). As is clear from the thus-obtained data, YK336, YK338, or YK339 exhibits, in cultured cells, growth potential comparable to that of the wild-type virus.
Vero cells were infected with YK381, YK382, or wild-type HSV-2 186 at MOI 0.01 or MOI 3, and the amount of the virus present inside and outside of the virus-infected cells was determined for comparison of growth potential (Fig. 5). As is clear from the thus-obtained data, YK381 or YK382 exhibits, in cultured cells, growth potential comparable to that of the wild-type virus.
YK336, YK338, YK339, or wild-type HSV-1(F) was inoculated into the brain of mice at different doses, and the LD50 of the virus was determined. As a result, no significant difference was observed in LD50 between these viruses (Table 1).
YK381, YK382, or wild-type HSV-2(186) was inoculated into the brain of mice at different doses, and the LD50 of the virus was determined. As a result, no significant difference was observed in LD50 between these viruses (Table 2).

**[Table 1]**

| Pathogenicity (LD50) upon inoculation into the brain of mice | |
|---|---|
| Virus | PFU/mL |
| HSV-1(F) | 10^{1.8} |
| YK336 | 10^{2.3} |
| YK338 | 10^{2.5} |
| YK339 | 10^{2.3} |

**[Table 2]**

| Pathogenicity (LD50) upon inoculation into the brain of mice | |
|---|---|
| Virus | PFU/mL |
| HSV-2(186) | 10^{-0.3} |
| YK381 | 10^{-0.5} |
| YK382 | 10^{0.1} |

As is clear from the aforementioned data, insertion of a foreign gene into a region between polyA signals of two genes encoded in directions opposing one another in the genome of HSV does not affect growth of the virus in cultured cells, or pathogenicity of the virus in a mouse model.

### Example 2

### (1) Construction of pBS246GFPBAC

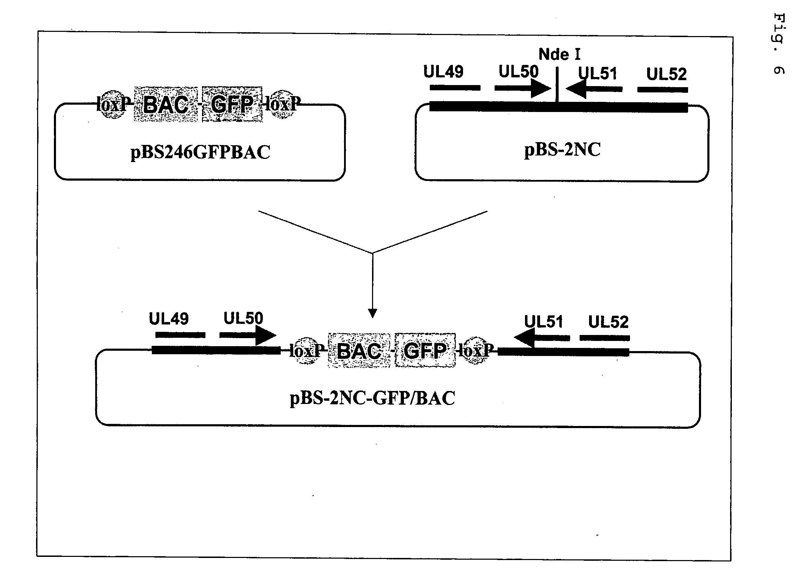

pEGFP-C1 (Clontech) was digested with BglII and BamHI, followed by blunt-ending and ligation. An AseI-AflIII fragment of the resultant product was blunt-ended and cloned into an SmaI site of pBS246 (Invitrogen). Furthermore, an SalI fragment of pBelloBAC11 (Research Genetics) was blunt-ended and cloned into EcoRV (Fig. 6).

### (2) Construction of pBS-2NC-EGFP/BAC

An NotI-ClaI fragment of the genomic DNA of HSV-2 186 strain (n.n. 106473 to n.n. 110443) was cloned into an NotI-ClaI site of pBliuescript II KS+ (Stratagene), to thereby produce pBS-2NC. An NdeI site of the resultant plasmid was blunted, and a blunt-ended NotI fragment of pBS246GFPBAC was cloned thereinto, to thereby construct pBS-2NC-EGFP/BAC (Fig. 6).

### (3) Construction of YK351

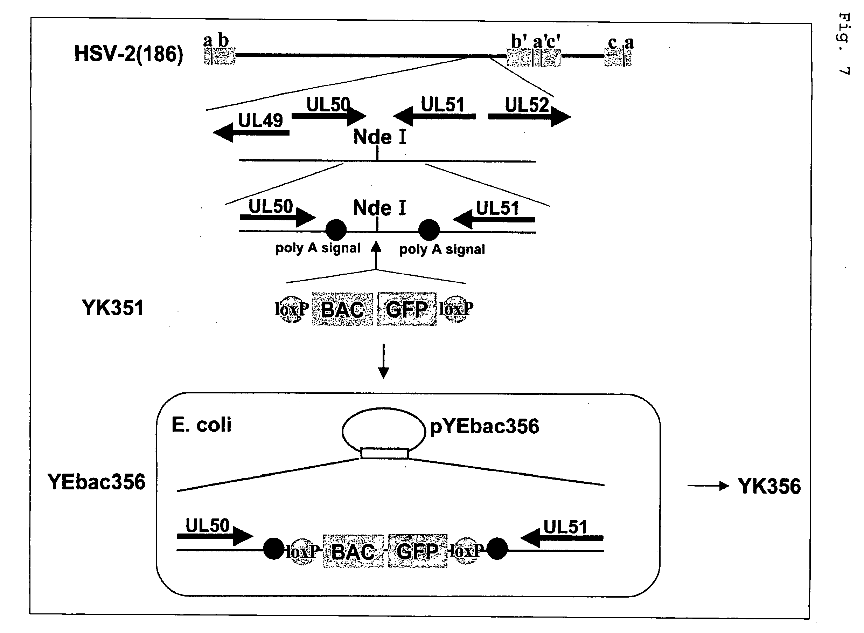

The thus-obtained pBS-2NC-EGFP/BAC and the virus DNA of HSV-2 186 strain were transfected into rabbit skin cells (RSC), followed by homologous recombination, to thereby produce a recombinant virus YK351 including an EGFP expression cassette and bacmid inserted in a region between the UL50 and UL51 genes. The recombinant virus was selected by collecting a fluorescent plaque under a fluorescence microscope. Plaque purification was carried out under a fluorescence microscope until 100% of the plaque fluoresced. YK351 was confirmed to be a recombinant virus of interest through Southern blotting. YK351 includes bacmid and the EGFP expression cassette sandwiched between loxP sequences and inserted into the UL50-UL51 intergenic region, and retains the full-length HSV-2 gene without deletion of any known HSV-2 ORF (Fig. 7).

### Example 3 (Construction of YEbac356)

Vero cells were infected with YK351, and circular virus DNA is extracted from the thus-infected cells through the Hirt method. The thus-extracted circular virus DNA was incorporated into *Escherichia coli* DH10B (Invitrogen) through electroporation, followed by selection in a chloramphenicol-containing medium, to thereby yield *Escherichia coli* YEbac356 including the genome of YK351 (Fig. 7).

### Example 4 (Analysis of property of recombinant virus (YK356) reconstructed from pYEbac356)

Infectious HSV DNA (pYEbac356) was extracted from the *Escherichia coli* YEbac356 through the alkaline SDS method, and pYEbac356 was transfected into RSC through the calcium phosphate method. As a result, an infectious HSV-2 recombinant (YK356) was found to be produced.
Fig. 8 shows the restriction enzyme (NotI) cleavage patterns, on agarose electrophoresis, of the recombinant virus (YK356) reconstructed from pYEbac356 and wild-type HSV-2 186 DNA. In-Fig. 8, "a" corresponds to a band observed through insertion of bacmid, and "b" corresponds to a band which disappears through insertion of bacmid. As shown in Fig. 8, the restriction enzyme cleavage pattern of YK356 is almost the same as that of wild-type HSV-2(186), excepting a bacmid insertion region.
Vero cells were infected with the thus-produced YK356 or wild-type HSV-2 186 at MOI 0.01 or MOI 3, and the amount of the virus present inside and outside of the virus-infected cells was determined for comparison of growth potential (Fig. 9). As is clear from the thus-obtained data, YK356 exhibits, in cultured cells, growth potential comparable to that of the wild-type virus.

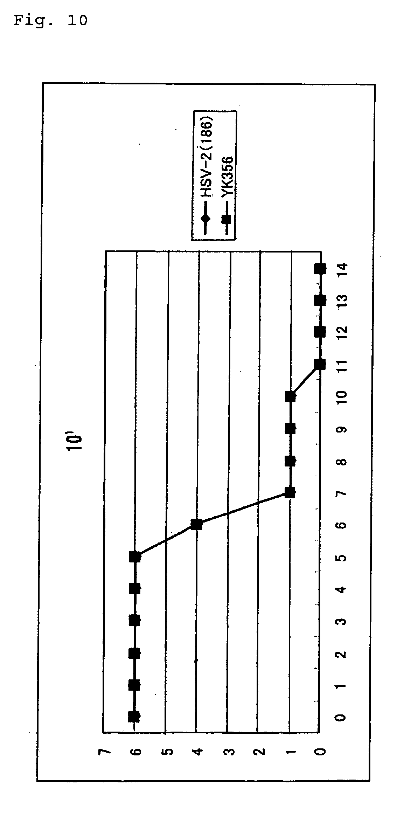

YK356 or wild-type HSV-2 186 was inoculated into the brain of ICR mice at 10 PFU, and the survival of the thus-infected mice was observed for two weeks. As a result, the survival curve of mice infected with YK356 was the same as that of mice infected with wild-type HSV-2 186 (Fig. 10). YK356 or wild-type HSV-2 186 was inoculated into the brain of mice at different doses, and the LD50 of the virus was determined. As a result, no significant difference was observed in LD50 between these viruses (Table 3).

**[Table 3]**

| Virus | PFU/mL |
|---|---|
| HSV-2 (186) | 10^{-0.5} |
| YK356 | 10^{-0.2} |

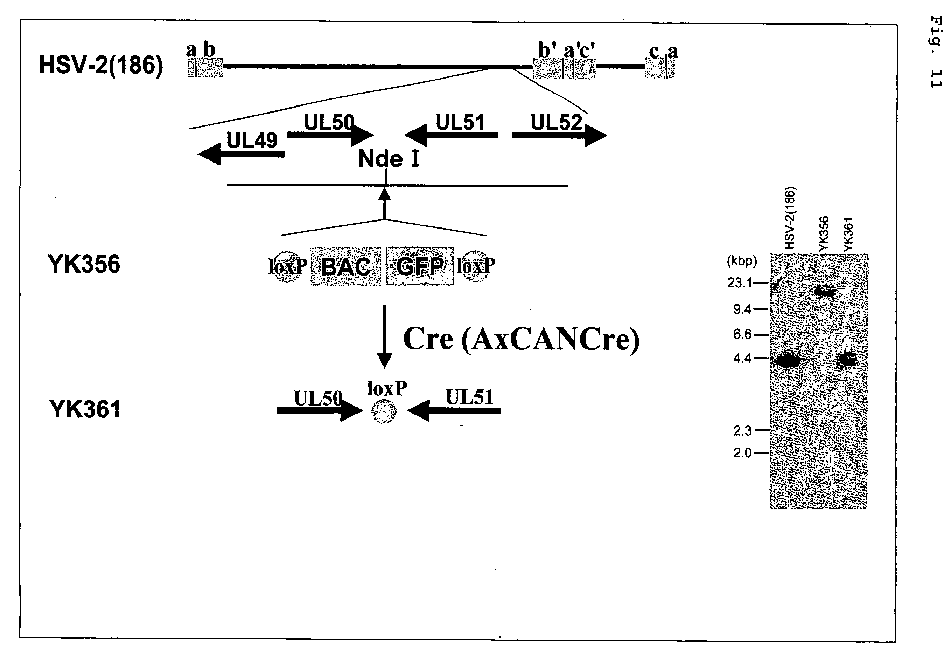

Vero cells were co-infected with YK356 and recombinant adenovirus AxCANCre which expresses Cre recombinase, to thereby make an attempt to remove bacmid (J. Virol. 77: 1382-1391, 2003). In the resultant recombinant virus YK361, bacmid was found to be removed from the genome of the virus through Southern blotting (Fig. 11). In Southern blotting, the virus DNA of HSV-2 186, YK356, or YK361 was employed in an experiment after treatment with NotI. The probe employed was a PCR-amplified region (500 bp) bridging between the UL50 and UL51 genes. The primers employed for PCR were as follows: acgcctgtgcgtttgttgta (SEQ ID NO: 1) and atggtcaacctcgagaggct (SEQ ID NO: 2).

### Example 5 (Mutation of the genome of HSV-2 in YEbac356)

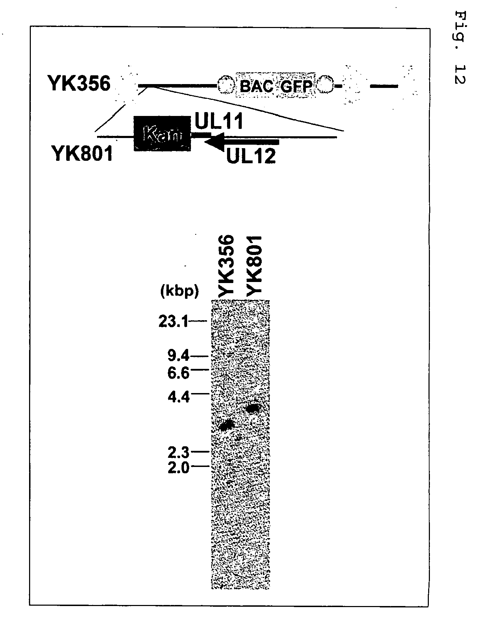

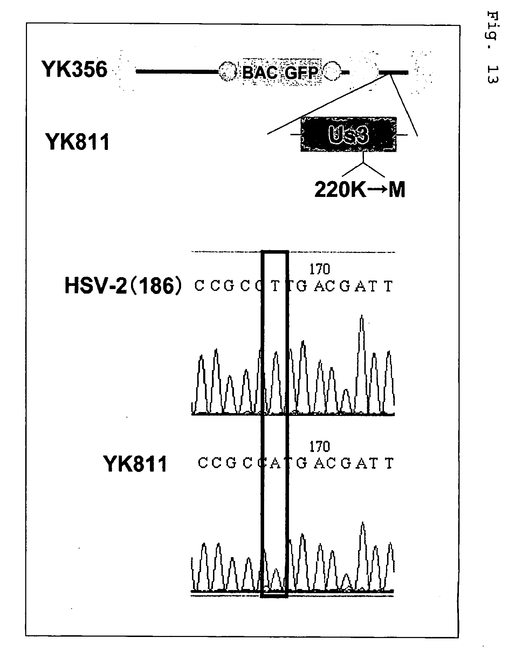

A kanamycin-resistant gene was inserted into the UL11 region of HSV-2 through the known method (J. Virol. 81: 10575-10587, 2007), to thereby make an attempt to inactivate the virus gene. Also, an attempt was made to replace Lys-220 of Us3 with methionine for mutation. After mutation of the genome of HSV-2 in *Escherichia coli* through the known method (J. Virol. 81: 10575-10587, 2007), the virus genome was extracted from *Escherichia coli,* and then transfected into RSC, to thereby produce a mutant virus (YK801: UL11 mutation or YK811: Us3 mutation). The primers employed for mutation were as follows.
UL11 mutation:
   caccgacggcggggaggtcgtctcgctgaccgcccactaatttgacgtcgtggacatcgaa ggatgacgacgataagtaggg (SEQ ID NO: 3), and
   taccctcttcttcggactcgatgtccacgacgtcaaattagtgggcggtcagcgagacgac aaccaattaaccaattctgattag (SEQ ID NO: 4).
Us3 mutation:
   tgatagcagccacccgaactaccctcatcgggtaatcgtcatggcggggtggtacgccaga ggatgacgacgataagtaggg (SEQ ID NO: 5), and
   gccgcgcctcgtggctcgtgctggcgtaccaccccgccttgacgattacccgatgagggtc aaccaattaaccaattctgattag (SEQ ID NO: 6).
For confirmation of mutation of UL11, the genome of the virus YK801 or YK356 was treated with BamHI and then subjected to Southern blotting. The probe employed was a PCR-amplified region (about 500 bp) bridging between the UL11 and UL12 genes. The primers employed were gtggtgtttattttcccccc (SEQ ID NO: 7) and tcgagccggggatctaccgg (SEQ ID NO: 8). For confirmation of mutation of Us3, the nucleotide sequence of the corresponding region in the genome of the virus YK811 was determined. As shown in Figs. 12 and 13, mutation of interest (SEQ ID NO: 9 or 10) was confirmed.

*Escherichia coli* YEbac356 including the full-length genome of HSV-2 was successfully constructed. A recombinant virus reconstructed from the virus genome (pYEbac356) retained in YEbac356 exhibited growth potential in cultured cells and pathogenicity in a mouse model comparable to those of the corresponding wild-type virus. Bacmid was readily removed from the virus genome by means of a Cre-loxP system. In addition, mutation of interest was readily carried out in YEbac356.

## Claims

1. A method for producing a recombinant virus comprising, inserting a foreign gene into a region between polyA signals of two genes encoded in directions opposing one another in the genome of a target virus, excepting a region between the UL3 and UL4 genes of HSV-1.

2. The method for producing the recombinant virus according to claim 1, wherein the foreign gene is a gene selected from among bacmid, a reporter gene, a gene encoding an immunogenic protein, and a gene used for gene therapy.

3. A method for producing an *Escherichia coli* including the genome of a recombinant virus which substantially retains the full-length genome of a wild-type virus comprising, inserting a bacmid into a region between polyA signals of two genes encoded in directions opposing one another in the genome of a target virus, excepting a region between the UL3 and UL4 genes of HSV-1, and incorporating the genome of the recombinant virus into an *Escherichia coli.*

4. The production method according to any one of claims 1 to 3, wherein the virus is a virus selected from among herpesvirus, adenovirus, poxvirus, and papovavirus.

5. A herpes simplex virus type 2 recombinant which substantially retains the full-length genome of a wild-type herpes simplex virus type 2, comprising a bacmid inserted into a region between the UL50 and UL51 genes in the full-length genome.

6. An *Escherichia coli* including the genome of a herpes simplex virus type 2 recombinant which substantially retains the full-length genome of wild-type herpes simplex virus type 2, and in which bacmid is inserted into a region between the UL50 and UL51 genes in the full-length genome.


SEQUENCE LISTING

<110> The University of Tokyo

<120> A Recombinant Virus, Escherichia colli having the virus genome and
a process for producing thereof

<130> TU0007 PCT

<150> JP2008-142662
<151> 2008-05-30

<160> 10

<170> PatentIn version 3.1

<210> 1
<211> 20
<212> DNA
<213> Artificial Sequence

<220>
<223> Designed primer based on HSV-2

<400> 1
acgcctgtgc gtttgttgta                                                    20

<210> 2

<211> 20

<212> DNA

<213> Artificial Sequence

<220>

<223> Designed primer based on HSV-2

<400> 2

atggtcaacc tcgagaggct 20

<210> 3

<211> 82

<212> DNA

<213> Artificial Sequence

<220>

<223> Designed primer based on HSV-2

<400> 3

caccgacggc ggggaggtcg tctcgctgac cgcccactaa tttgacgtcg tggacatcga 60

aggatgacga cgataagtag gg 82

<210> 4

<211> 85

<212> DNA

<213> Artificial Sequence

<220>

<223> Designed primer based on HSV-2

<400> 4

taccctcttc ttcggactcg atgtccacga cgtcaaatta gtgggcggtc agcgagacga 60

caaccaatta accaattctg attag 85

<210> 5

<211> 82

<212> DNA

<213> Artificial Sequence

<220>

<223> Designed primer based on HSV-2

<400> 5

tgatagcagc cacccgaact accctcatcg ggtaatcgtc atggcggggt ggtacgccag 60

aggatgacga cgataagtag gg 82

<210> 6
<211> 85
<212> DNA
<213> Artificial Sequence

<220>
<223> Designed primer based on HSV-2

<400> 6
gccgcgcctc gtggctcgtg ctggcgtacc accccgcctt gacgattacc cgatgagggt 60

caaccaatta accaattctg attag 85

<210> 7
<211> 20
<212> DNA
<213> Artificial Sequence

<220>
<223> Designed primer based on HSV-2

<400> 7
gtggtgttta ttttcccccc 20

<210> 8

<211>  20
<212>  DNA
<213>  Artificial Sequence

<220>
<223>  Designed primer based on HSV-2

<400>  8
tcgagccggg gatctaccgg                                                    20

<210>  9
<211>  61
<212>  DNA
<213>  UL11 gene in HSV-2

<400>  9
gcgcctcgtg gctcgtgctg gcgtaccacc ccgccttgac gattacccga tgagggtagt      60

t                                                                        61

<210>  10
<211>  61
<212>  DNA
<213>  Variant of UL11 gene in HSV-2

<400> 10
gcgcctcgtg gctcgtgctg gcgtaccacc ccgccatgac gattacccga tgagggtagt 60
t 61